# FASCICULE DE BREVET EUROPEEN

(11) **EP 4 106 681 B1**
(45) Date de publication et mention de la délivrance du brevet: **28.05.2025**
(21) Numéro de dépôt: 21709081.0
(22) Date de dépôt: 11.02.2021
(51) Int. Cl.: A61F 2/50, A61F 2/54, A61F 2/60

(54) **PROTHÈSE DE MEMBRE**
SCHENKELPROTHESE
LIMB PROSTHESIS

(30) Priorité: 19.02.2020 FR 2001628
(43) Date de publication de la demande: 28.12.2022
(73) Titulaire: Chabloz Orthopédie, 38170 Seyssinet-Pariset (FR)
(72) Inventeur: SOUPLY, Marc, 38220 VIZILLE (FR); KHOUNLAVONG, Anousak, 38170 SEYSSINET-PARISET (FR); RONAYETTE-LAMOINE, Elodie, 38100 GRENOBLE (FR); REVAIS, Jules, 38170 SEYSSINET-PARISET (FR)
(74) Mandataire: Brevalex
(86) Numéro de dépôt international: PCT/FR2021/050248
(87) Numéro de publication internationale: WO 2021/165601

(56) Documents cités:
- WO-A1-2017/012888
- DE-C- 322 909
- KR-A- 20110 034 879
- NICHOLAS HERBERT ET AL: "A preliminary investigation into the development of 3-D printing of prosthetic sockets", JOURNAL OF REHABILITATION RESEARCH AND DEVELOPMENT, vol. 42, no. 2, 1 January 2005 (2005-01-01), US, pages 141, XP055293669, ISSN: 0748-7711, DOI: 10.1682/JRRD.2004.08.0134

## Description

### DOMAINE TECHNIQUE ET ÉTAT DE LA TECHNIQUE ANTÉRIEURE

La présente invention se rapporte à une structure exosquelettique d'une prothèse d'un membre donné.

Suite à une amputation d'un membre, pour retrouver sa mobilité, un patient se trouve habituellement appareillé avec une prothèse qui va partiellement remplacer la fonctionnalité du membre perdu. La forme physique d'une telle prothèse est déterminée par les contraintes imposées par sa fonction. Par exemple, pour obtenir une prothèse de remplacement de la jambe, la prothèse connue de l'état de l'art comprend une barre se terminant par une articulation mécanique afin d'assumer aussi bien que possible une fonctionnalité de jambe. Afin de conférer une esthétique similaire au membre perdu, une telle prothèse connue de l'art antérieur peut être parée d'une enveloppe d'habillage, également connue de l'art antérieur. Un exemple d'une telle enveloppe est décrit par le document EP 2 944 290 A1. Pour obtenir un aspect naturel, l'enveloppe décrite par le document cité emprunte la forme d'une jambe. De plus, des ouvertures sont prévues pour permettre une aération et pour réduire le poids de l'enveloppe. WO 2017/012888 A1 décrit une structure exosquelettique suivant le préambule de la revendication 1.

Les enveloppes connues dans l'art antérieur ne reproduisent qu'imparfaitement un membre remplacé. La nécessité de manipuler une prothèse et une enveloppe peut s'avérer encombrant.

### EXPOSÉ DE L'INVENTION

Le but de la présente invention est alors d'offrir une prothèse d'un membre donné qui reproduit de manière plus réaliste le membre remplacé.

Le but énoncé ci-dessus est atteint par une structure exosquelettique de la prothèse qui ressemble, au toucher, au membre remplacé. La structure exosquelettique de la présente invention ressemble par sa flexibilité aux différentes parties ressenties en touchant le membre remplacé. Ladite structure exosquelettique est conçue pour assurer une liaison entre une emboîture et une prothèse de main ou de pied afin de former la prothèse du membre remplacé.

Suivant l'invention, la structure exosquelettique de prothèse d'un membre donné comprend au moins deux zones de flexibilité différentes, l'agencement desdites zones entre elles correspondant à l'agencement de parties du membre donné ayant des duretés différentes.

D'autres modes de réalisation sont montrés dans les revendications 2-15.

### BRÈVE DESCRIPTION DES DESSINS

La présente invention sera mieux comprise sur la base de la description qui va suivre et des dessins en annexe sur lesquels :
[Fig. 1] montre un premier exemple d'une prothèse comprenant une structure exosquelettique selon une première vue,
[Fig. 2] montre une anatomie d'un membre donné selon une première vue,
[Fig. 3] montre un premier exemple d'une prothèse comprenant une structure exosquelettique selon une deuxième vue,
[Fig. 4] montre une anatomie d'un membre donné selon une deuxième vue,
[Fig. 5] montre une vue en coupe longitudinale d'une prothèse selon le premier exemple comprenant une structure exosquelettique,
[Fig. 6] montre un deuxième exemple d'une prothèse comprenant une structure exosquelettique,
[Fig. 7] montre une anatomie d'un autre membre donné,
[Fig. 8] montre une vue en coupe longitudinale d'une prothèse selon le deuxième exemple comprenant une structure exosquelettique,
[Fig. 9] montre une vue en coupe longitudinale d'une prothèse selon un troisième exemple comprenant une structure exosquelettique,
[Fig. 10] montre une vue en coupe d'une prothèse selon un quatrième exemple comprenant une structure exosquelettique,
[Fig. 11] montre une vue en coupe d'une structure exosquelettique d'une prothèse selon un cinquième exemple.

### EXPOSÉ DÉTAILLÉ DE MODES DE RÉALISATIONS PARTICULIERS

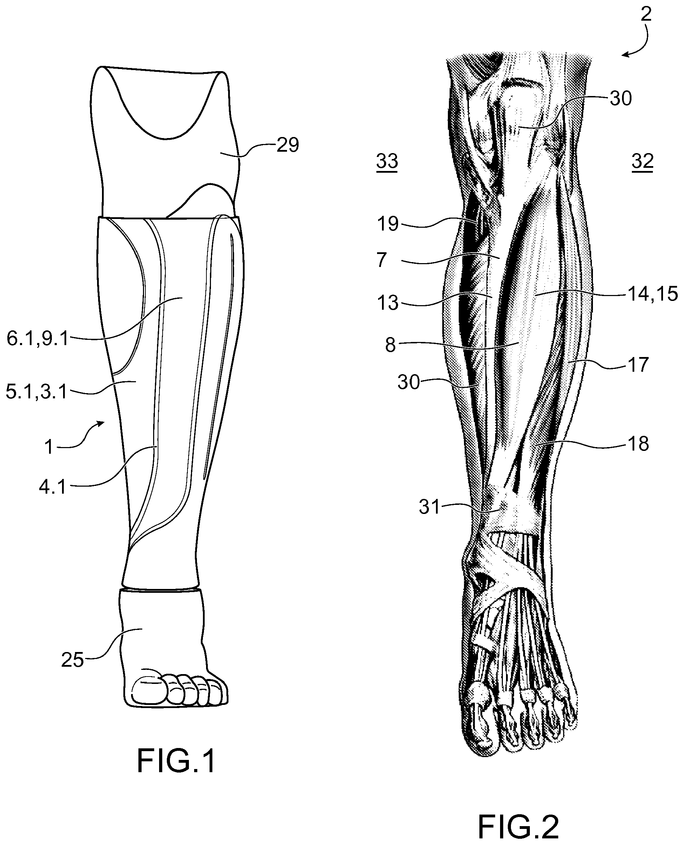

Un exemple de réalisation d'une structure exosquelettique d'une prothèse est illustré en figures 1, 3, 5, 6 et 8 à 10.

Les figures 2, 4 et 7 montrent une anatomie d'un membre donné. Dans l'exemple présent, il s'agit de la partie inférieure (tibiale) d'une jambe ou d'un avant bras.

Sur lesdites figures on identifie des parties de la jambe ou du bras ayant des duretés différentes (7, 8).

Entre le genou (30) et la cheville (31) s'étend le tibia (13), qui est l'un des os le plus important de ce membre inférieur. On observe également le tibial antérieur (14), qui est un des muscles (15) de la partie tibiale de la jambe humaine. Dans un bras, le radius et l'ulna s'étendent entre le poignet et le coude et assurent ainsi une liaison entre la main et le bras supérieur. On observe également le flexor, qui est un muscle proéminent du bras.

Le tibia (13) est situé sous une peau (non montré) de la jambe. Cet os se prolonge presque en ligne droite entre le genou et la cheville. Entre le tibia et la peau se situe le tibial antérieur (14). Ce muscle se prolonge, sous la peau, d'un côté extérieur (32) du genou jusqu'à un côté intérieur (33) de la cheville et traverse le tibia. On peut ainsi identifier une partie où le tibia se trouve directement sous la peau et une partie où le tibial antérieur se trouve directement sous la peau, entre la peau et le tibia.

En touchant la peau de la jambe on peut ainsi distinguer une première partie de dureté (7) et une deuxième partie de dureté (8), c'est à dire des parties du membre ayant des duretés différentes. De la même façon on identifie sur un bras des parties de duretés différentes correspondant par exemple à l'ulna (os) et le flexor (muscle).

Au toucher, une partie sera ressentie comme molle si un muscle ou un tendon se trouve directement sous la peau. Une partie sera ressentie comme dure si un os se trouve directement sous la peau.

Dans le présent exemple, la partie de la jambe ou du bras où le tibia ou l'ulna se trouve directement sous la peau est une partie ressentie comme dure de, la partie de la jambe ou du bras où le tibial antérieur ou le flexor se trouve directement sous la peau est une partie ressentie comme molle. On identifie ainsi deux parties ayant des duretés différentes.

Touché de façon plus soigneuse, on peut différencier encore plus que deux parties de duretés différentes. Par exemple, une partie ayant plus de tissu musculaire entre la peau et le tibia sera plus dure qu'une partie ayant moins de tissu musculaire entre la peau et le tibia. Une partie où se trouve un tendon sera ressentie comme plus dure qu'une partie où se trouve un muscle, mais moins dure qu'une partie où se trouve un os.

D'autres parties molles et dures de la jambe sont formées par la position des autres os, muscles et tendons sur la jambe, comme le long fibulaire (17), le long extenseur des orteils (18), le gastrocnémien (19) ou le tendon d'Achille (20). On identifie ainsi une pluralité de parties de la jambe ayant des duretés différentes.

Des parties d'un membre ayant des duretés différentes ont été exemplifiées pour une jambe inférieure, mais elles peuvent également être retrouvées sur la partie supérieure de la jambe, sur le bras et à d'autres endroits du corps.

L'agencement des parties du membre ayant des duretés différentes, c'est à dire la délimitation d'une zone dure ou molle et la position relative d'une zone dure par rapport à une zone molle dépend de l'anatomie du membre choisi. Cet agencement est une caractéristique du membre donné qui est remplacé par la prothèse.

Le présent exemple de réalisation porte sur une structure exosquelettique d'une prothèse d'un membre et plus particulièrement une structure exosquelettique d'une prothèse d'un bras ou d'une jambe. L'invention s'applique pourtant de la même façon à d'autres parties du corps présentant des duretés différentes.

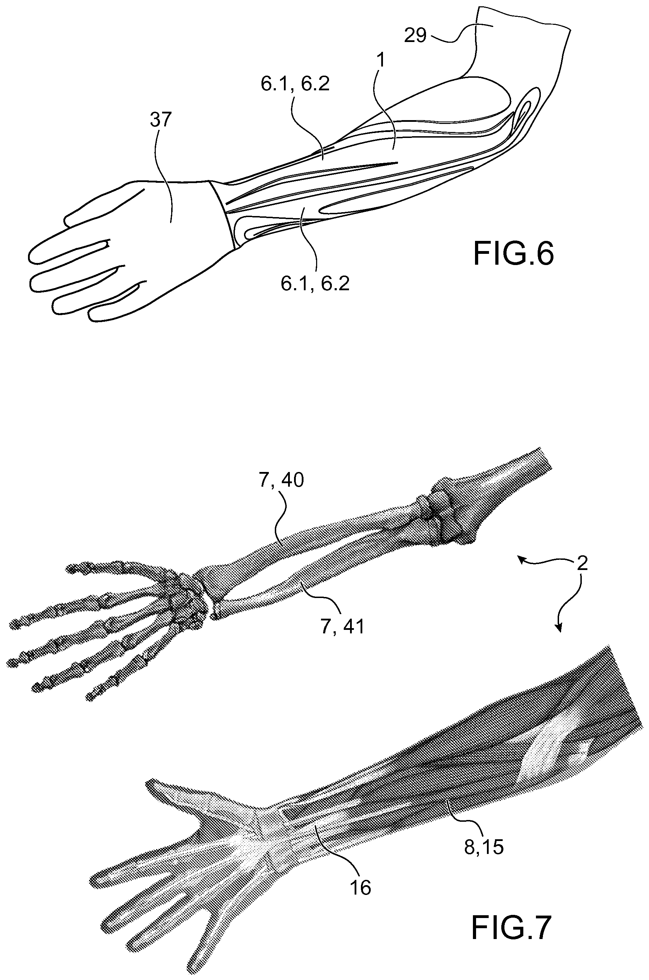

Les figures 1, 3 et 5 montrent une prothèse (12) de jambe. Les figures 6, 8, 9, 10 et 11 montrent une prothèse de bras.

Sur les figures 1, 3, 5, 6, et 9 on identifie une emboîture (29). L'emboîture (29) est adaptée pour se fixer sur un moignon de bras pour une prothèse de bras ou sur un moignon de jambe pour une prothèse de jambe. L'emboîture assure ainsi la fixation de la prothèse au corps. On identifie également une prothèse de pied (25) et une prothèse de main (37). La prothèse de pied et la prothèse de main servent un patient à effectuer les tâches effectuées par un pied ou une main d'un membre naturel. Plus particulièrement, la prothèse de pied peut supporter un poids du corps et assurer un mouvement durant la marche. La prothèse de main peut, par exemple, soulever un poids, ou pousser un objet. Dans les deux cas, une force doit être transmise entre la prothèse de pied ou la prothèse de main et l'emboîture pour assurer un mouvement. De la même façon, une force doit être transmise si un utilisateur se pose sur la prothèse de pied ou la prothèse de main.

On identifie également une structure exosquelettique de la prothèse (1). La structure exosquelettique assure une liaison entre l'emboîture (29) et la prothèse de pied (25) ou la prothèse de main (25). La structure exosquelettique est posé entre l'emboîture et la prothèse de pied ou la prothèse de main. D'un côté de la structure exosquelettique peut être fixé l'emboîture. De l'autre côté du rapport peut être fixé la prothèse de pied ou de main.

La structure exosquelettique assure la liaison entre l'emboîture et la prothèse de pied ou de main en permettant la transmission de force pour assurer le mouvement de la prothèse de pied ou de main. De la même façon, la structure exosquelettique assure la transmission de force entre l'emboîture et le pied prothétique ou la main prothétique si l'utilisateur se pose sur le pied prothétique ou sur la main prothétique.

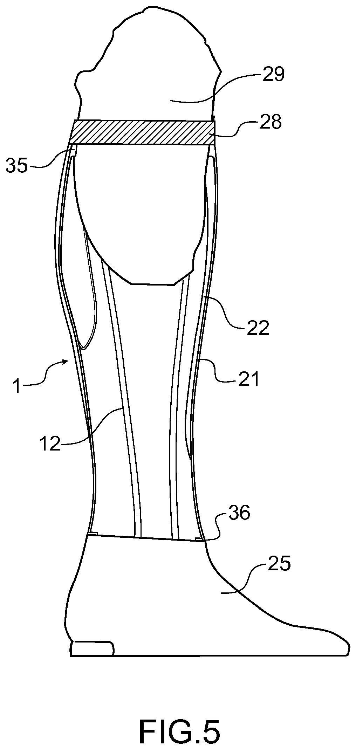

La structure exosquelettique peut avoir une forme tubulaire. Par forme tubulaire on comprend une forme cylindrique qui peut présenter des coupes ayant une forme et une taille variable. La forme tubulaire peut également être courbée et ainsi suivre majoritairement un axe non droit. La structure exosquelettique montrée dans la figure 5 présente ainsi une coupe de forme et de taille qui est différent à un niveau du mollet et à un niveau de la cheville. La structure exosquelettique de forme tubulaire pourrait également être courbée et ainsi suivre une emboîture (29) qui présente un angle par rapport à la prothèse (12). Une structure exosquelettique qui a une forme de jambe ou de bras présente ainsi une forme tubulaire.

Les figures 5, 8 et 9 montrent une surface intérieure (22) et une surface extérieure (21) de la structure exosquelettique. L'épaisseur de la structure exosquelettique est une quantité de matériau compris entre ladite surface intérieure et surface extérieure de la structure exosquelettique. On identifie sur les figures 1, 3 et 6 également une première épaisseur (3.1, 3.2, 3.3) de la structure exosquelettique, une deuxième épaisseur (9.1, 9.2) et un changement d'épaisseur (4.1, 4.2) situé entre la première (3.1, 3.2, 3.3) et la deuxième (9.1, 9.2) épaisseur.

Le changement d'épaisseur délimite ainsi une première zone (5.1, 5.2) ayant la première épaisseur (3.1, 3.2, 3.3) d'une deuxième zone (6.1, 6.2) ayant la deuxième épaisseur (9.1, 9.2).

La structure exosquelettique présente une flexibilité en dépendance de son épaisseur. Ainsi, si la première zone (5.1, 5.2) a une épaisseur plus importante que la deuxième zone (6.1, 6.2), la première zone (5.1, 5.2) aura une flexibilité moins importante que la deuxième zone (6.1, 6.2).

Autrement dit, si la première zone (5.1, 5.2) à une épaisseur plus importante qu'une épaisseur moyenne de la structure exosquelettique et si la deuxième zone (6.1, 6.2) a une épaisseur moins importante qu'une épaisseur moyenne de la structure exosquelettique, la première zone (5.1, 5.2) aura une flexibilité moins importante que la deuxième zone (6.1, 6.2).

Des zones de flexibilité différentes peuvent aussi être obtenues par une différence de matériau. Une première zone peut être fabriquée d'un premier matériau, une deuxième zone peut être fabriquée d'un deuxième matériau. Le premier matériau peut être moins flexible que le deuxième matériau. On obtient ainsi deux zones de flexibilité différentes.

Une différence de matériau peut être comprise comme une différence de composition chimique du matériau. Ainsi, un polymère utilisé peut être plus flexible s'il comprend une composante chimique ajoutée, par exemple s'il comprend un plastifiant.

Une différence de matériau peut également être compris comme une différence en composition macroscopique. Ainsi, une deuxième zone peut comprendre un polyuréthane thermoplastique. Une première zone peut comprendre le polyuréthane thermoplastique et aussi des fibres de carbone. On obtient ainsi deux zones de flexibilité différentes, la première zone ayant une flexibilité moins importante que la deuxième zone à cause des propriétés des fibres de carbone.

Il est également possible d'obtenir des zones de flexibilité différentes par une différence de motif en trois dimensions imprégné sur la zone. Par exemple, une deuxième zone peut comprendre un polyuréthane thermoplastique. Une première zone peut comprendre l'uréthane thermoplastique imprégné d'une déformation en trois dimensions rendant ladite zone moins flexible.

En touchant la structure exosquelettique, la zone d'une flexibilité plus importante donne l'impression de toucher une partie molle d'un membre alors que la zone de flexibilité moins importante donne l'impression de toucher une partie dure d'un membre.

La structure exosquelettique peut aussi comprendre une pluralité de zones présentant des flexibilités différentes. On peut prévoir deux, trois, quatre ou plus de zones, l'ensemble des zones présentant deux, trois ou plus de flexibilités différentes. Par exemple, on peut prévoir cinq zones, l'ensemble des cinq zones ayant trois flexibilités différentes.

De cette façon, il est possible d'obtenir une pluralité de flexibilités différentes. On peut ainsi obtenir une structure exosquelettique qui fait sentir, au toucher, une pluralité de duretés différentes. De cette façon, la structure exosquelettique peut encore plus précisément ressembler à un toucher d'une anatomie d'un membre parce que, touché de façon plus soigneuse, on peut différencier plus de deux parties de duretés différentes sur l'anatomie d'un membre. Il est également possible de varier une combinaison d'une épaisseur, d'un matériau et/ou d'une structure en trois dimensions pour obtenir des zones de flexibilité différentes.

De manière générale, les zones les plus flexibles peuvent correspondre à un tissu musculaire. Pour une structure exosquelettique d'une prothèse de bras, il s'agit par exemple des muscles comme le brachioradial, le groupe des flechisseurs du poignet ou le groupe radial. Des zones moins flexibles peuvent correspondre à des parties fibreuses du bras, par exemple les tendons des muscles précédents, les muscles des doigts ou les ligaments du poignet. Les zones encore moins flexibles ou durs peuvent correspondre aux os, par exemple le radius ou l'ulna, de préférence principalement dans les zones articulaires.

De différentes épaisseurs de la structure exosquelettique peuvent être fabriquées en faisant varier une épaisseur d'un matériau utilisé pour une fabrication de la structure exosquelettique. Il est également possible d'utiliser un matériau comprenant plusieurs couches et de varier d'un endroit à un autre endroit le nombre de couches pour arriver à l'épaisseur différente de la structure exosquelettique. Il est également possible d'utiliser des couches faites de différents matériaux.

La structure exosquelettique assure la liaison entre l'emboîture (29) et la main prothétique (37) ou le pied prothétique (25) et permet ainsi la transmission de force entre la main ou le pied prothétique et l'emboîture.

De façon avantageuse ladite liaison est majoritairement assurée par la première zone seule. Autrement dit, la transmission de ladite force est assurée majoritairement par la première zone seule. La première zone toute seule peut ainsi assurer la transmission de la totalité de ladite force. Ceci signifie que la liaison entre l'emboîture et la main ou le pied prothétique reste assurée même si la deuxième zone était complètement enlevée de la structure exosquelettique.

Comme décrit ci-dessus, la première zone peut posséder une flexibilité moins importante que la deuxième zone. Ayant une flexibilité moins importante, la première zone se déforme moins sous l'effet d'une force donnée qu'une deuxième zone, qui possède une flexibilité plus importante. Autrement dit, pour un degré de déformation donné, la première zone peut supporter une force plus élevée que la deuxième zone.

Dans le cas où ladite liaison est majoritairement assurée par la première zone seule, la force transmise durant une utilisation habituelle entre l'emboîture et la main ou le pied prothétique résulte en une déformation d'une grandeur acceptable de la première zone si la deuxième zone était enlevée de la structure exosquelettique.

En comparant la structure exosquelettique de la figure 1 avec l'anatomie de la figure 2 on observe que l'agencement des zones de flexibilité différentes (5.1, 5.2, 6.1, 6.2) entre elles correspond à l'agencement des parties du membre ayant des duretés différentes (13, 14, 17).

On observe sur la figure 1 que la deuxième zone (6.1) est agencée de façon majoritairement longitudinale en se courbant vers le bas. La première zone (5.1) est agencée de façon à s'élargir d'un haut vers un bas.

La première zone (5.1) est ainsi agencée par rapport à la deuxième zone (6.1) sur la structure exosquelettique comme le tibia (13) est agencé par rapport au tibial antérieur (14).

On observe le même agencement des zones de flexibilité différentes entre elles en comparant la structure exosquelettique d'une prothèse de bras (figure 6) avec l'anatomie d'un bras (figure 7).

L'agencement des zones varient d'un patient à un autre, comme la position des os, muscles et tendons varie d'un patient à un autre.

Dans l'exemple présent, la première zone (5.1) a une épaisseur plus importante que la deuxième zone (6.1). Ladite première zone donne ainsi au toucher l'impression d'être plus dure que la deuxième zone. La deuxième zone donne l'impression d'être plus molle que la première zone.

En touchant la structure exosquelettique, l'utilisateur reçoit ainsi une sensation similaire à toucher une partie inférieure d'une jambe humaine ou un bras. Dans le cas d'une prothèse de jambe, la première zone (5.1) sera ressentie comme en touchant le tibia et la deuxième zone comme en touchant le muscle tibial antérieur. En glissant un doigt entre la première zone (5.1) et la deuxième zone (6.1), le changement de flexibilité, provoqué par le changement d'épaisseur de la structure exosquelettique, est ressenti comme un changement de fermeté, entre une partie molle et une partie dure d'une jambe humaine ou d'un bras humain.

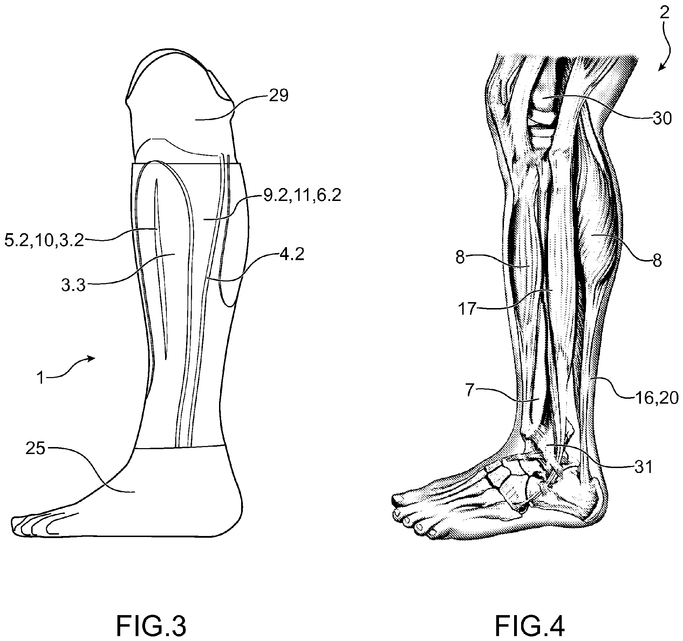

De la même façon, on observe sur les figures 3 et 4 qu'une deuxième zone (6.2) ayant une épaisseur moins importante est agencée sur la structure exosquelettique comme le long fibulaire (17) sur la partie tibiale d'une jambe.

Un changement d'épaisseur pour délimiter des zones de flexibilités différentes peut aussi être obtenu par une nervure ou par une échancrure sur la structure exosquelettique.

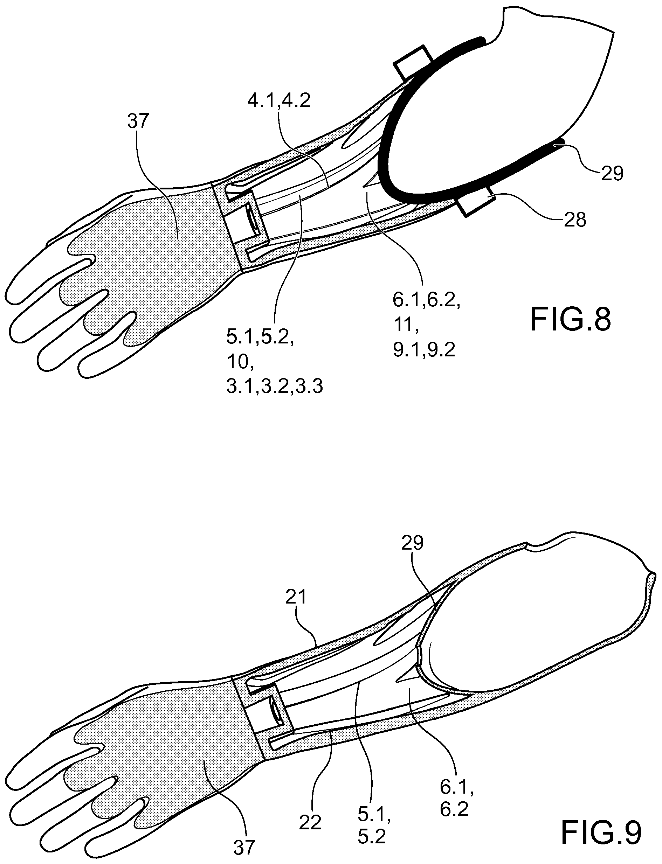

La figure 3 et la figure 8 montrent une nervure (10) sur la structure exosquelettique pour obtenir une flexibilité moins importante à l'endroit où se trouve la nervure. Les figures montrent également une échancrure (11) pour obtenir une flexibilité augmentée à l'endroit où se trouve l'échancrure.

Une nervure où une échancrure présente un changement d'épaisseur de la structure exosquelettique. Une structure exosquelettique ayant une nervure comprend une première zone et une deuxième zone ayant des épaisseurs différentes. On peut, par exemple, identifier comme première zone d'épaisseur plus importante la zone de la nervure elle-même. La deuxième zone d'épaisseur moins importante par rapport à la première zone est, dans ce cas, la zone qui se trouve en dehors de l'emplacement de la nervure.

Une utilisation de nervures ou d'échancrures permet un agencement particulièrement fin de zones de flexibilité différentes.

De façon avantageuse, la structure exosquelettique comprend un polyuréthane thermoplastique (thermoplastic polyurethane, TPU) et/ou un polyamide.

Le polyuréthane thermoplastique présente des avantages pour la fabrication d'une structure exosquelettique d'une prothèse. Il permet une bonne imitation d'un membre controlatéral, ce matériau peut aller dans l'eau et peut être lavé facilement. Sous un choc, la structure exosquelettique comprenant ce matériau se déforme et ne fait pas de bruit. Du fait de ses propriétés d'élasticité et de flexibilité, le TPU est particulièrement bien adapté pour la fabrication de zones de flexibilité différentes pour imiter un toucher physiologique. Une structure exosquelettique comprenant ce matériau est léger et ne rajoute pas trop de poids à la prothèse.

La structure exosquelettique peut être fabriqué par un procédé d'impression 3D, par exemple par fusion de poudre.

La première zone (5.1, 5.2) et la deuxième zone (6.1, 6.2) peuvent avoir une forme longitudinale (figure 1, 2) sur la structure exosquelettique, la forme longitudinale se prolongeant le long d'une extension du membre.

Dans l'exemple de réalisation montré aux figures 1, 3, 5, 6, 8, 9, 10 et 11, la structure exosquelettique a une forme tubulaire. La première épaisseur (3.1, 3.2, 3.3) et la deuxième épaisseur (9.1, 9.2) restent majoritairement constantes le long d'une longueur de la structure exosquelettique. L'épaisseur présente un changement majoritairement le long d'une circonférence de la forme tubulaire de la structure exosquelettique.

Dans le cas particulier de nervures ou d'échancrures, lesdites nervures et échancrures se prolongent majoritairement le long d'une longueur de la forme tubulaire.

Un agencement tel que décrit ci-dessus correspond particulièrement bien pour la fabrication d'une structure exosquelettique d'une prothèse pour un bras ou pour une jambe.

Les muscles, tendons et les os du bras et de la jambe sont allongés majoritairement le long d'une longueur du bras ou de la jambe. Pour cette raison, en touchant un bras ou une jambe, on ressent peu de changements de dureté en se promenant le long du membre. Une nervure ou une échancrure prévue le long d'une longueur de la forme tubulaire provoque un changement de dureté ressenti si un utilisateur promène son doigt sur la circonférence de la structure exosquelettique, la dureté restant constant le long de la longueur de la forme tubulaire. Au toucher, la structure exosquelettique reproduit ainsi un comportement similaire à une jambe ou à un bras. De façon très avantageuse, la première zone de la structure exosquelettique, qui provoque l'impression de dureté, similaire à un os, fournit également la stabilité à la prothèse, comme un os le fait dans un bras naturel. Comme un os assure la liaison entre la main et une partie supérieure du bras, la première zone assure de façon majoritaire la liaison entre la prothèse de main ou de pied et l'emboîture, qui repose sur un moignon du membre.

Les figures 5, 8 et 9 montrent une prothèse de jambe et une prothèse de bras. La prothèse comprend un pied prothétique (25) ou une main prothétique (37) et une emboîture (29). La structure exosquelettique (1) est située entre l'emboîture (29) et le pied prothétique (25) ou la main prothétique (37) et assure ainsi une liaison afin de former la prothèse de la jambe ou du bras.

On note également qu'aucun autre élément de support, autre que ladite structure exosquelettique, est prévu entre l'emboîture et le pied prothétique ou la main prothétique. Ainsi, la structure exosquelettique seul assure une liaison entre l'emboîture et le pied prothétique ou la main prothétique. La structure exosquelettique seul assure une transmission de force entre la main prothétique ou le pied prothétique et l'emboîture. De façon avantageuse, la première zone de la structure exosquelettique assure seule, de façon majoritaire, ladite liaison sans aucun autre élément de support prévu à l'intérieur de la structure exosquelettique.

La structure exosquelettique a une forme tubulaire (3) comprenant une surface intérieure (22) et une surface extérieure (21). De façon avantageuse, la surface extérieure (21) de la structure exosquelettique est majoritairement lisse par rapport aux changements (4.1, 4.2) délimitant des zones de flexibilité différentes.

La dilatation ou la rétractation du matériau qui forme une épaisseur plus importante (par exemple une nervure) ou moins importante (par exemple une échancrure) a lieu vers un intérieur de la structure exosquelettique (figure 5). La surface reste, de cette façon, lisse.

Plus précisément, la surface intérieure (22) s'éloigne ou se rapproche de la surface extérieure (21) pour provoquer un changement de l'épaisseur. L'ensemble de la surface extérieure et de la surface intérieure, formant une paroi de la structure exosquelettique, peut épouser une forme ressemblant à une anatomie d'un membre. On observe, par exemple sur la figure 5, que la paroi entre la surface extérieure (21) et la surface intérieure (22) se courbe d'un haut vers un bas pour faire ressembler la structure exosquelettique, à cet endroit, à un mollet. On note également sur la figure 6 que le diamètre de la structure exosquelettique diminue d'abord, en s'éloignant d'une position du poignet, puis adopte une forme arquée pour imiter une taille d'un muscle, avant de se terminer vers une position du coude. Une surface extérieure lisse signifie que la surface intérieure, tout en suivant la forme de la surface extérieure, s'éloigne et se rapproche de la surface extérieure pour former des zones d'épaisseur différentes. Dans le cas particulier de nervures (10) et/ou d'échancrures (11), majoritairement lisse signifie que la nervure et/ou l'échancrure est situé à l'intérieur de la structure exosquelettique.

La structure exosquelettique confère par son extérieur lisse encore plus un toucher physiologique, correspondant à une peau lisse ayant des duretés différentes au toucher.

Il est également possible de prévoir une surface intérieure majoritairement lisse et de situer les changements (4.1, 4.2) sur une surface extérieure. Ainsi, les nervures et/ou les échancrures peuvent être orientés vers une surface extérieure.

Aussi, les changements (4.1, 4.2) peuvent être situés sur la surface extérieure et sur la surface intérieure.

Selon un premier mode de réalisation, la structure exosquelettique (1), l'emboîture (29) et la prothèse de pied (25) ou la prothèse de main (37) sont prévus en trois parties séparées. Dans ce cas, la prothèse de pied ou de main est fixé par un premier côté de la structure exosquelettique. La structure exosquelettique est fixé par un deuxième côté sur l'emboîture. Les figures 5 et 8 montrent une structure exosquelettique (1) une prothèse de pied ou de main et une emboîture prévus en trois parties séparées. La structure exosquelettique peut être fixé sur l'emboîture par un collet de serrage (28). Le collet de serrage fixe la structure exosquelettique (1) sur l'emboîture de prothèse (29).

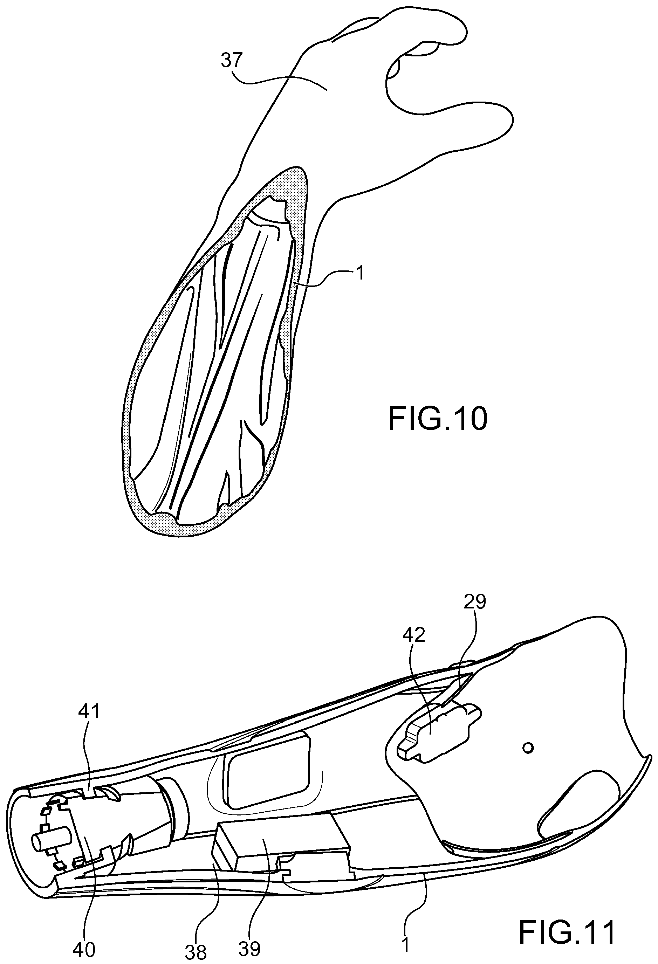

Selon un deuxième mode de réalisation, la structure exosquelettique peut être venue de matière avec la prothèse de pied ou la prothèse de main. Selon ce mode de réalisation, la structure exosquelettique forme un élément de matière continue avec la prothèse de pied ou de main. La figure 10 montre une structure exosquelettique formé en un seul élément de matière continue avec la prothèse de main.

Selon un troisième mode de réalisation, la structure exosquelettique peut être venu de matière avec l'emboîture. La structure exosquelettique forme ainsi un élément de matière continue avec l'emboîture. La figure 9 montre une structure exosquelettique venu de matière avec une emboîture (29), formant un seul élément de matière continue.

Selon un quatrième mode de réalisation, la structure exosquelettique, la prothèse de main ou la prothèse de pied et l'emboîture peuvent tous ensemble être venus de matière pour former un élément de matière continue. La structure exosquelettique, la prothèse de main ou la prothèse de pied et l'emboîture forment de cette façon un seul élément de matière continue.

La structure exosquelettique, l'emboîture et la prothèse de pied ou de main décrits ci-dessus peut être fabriqué avantageusement par le procédé décrit par la suite.

Dans une première étape, une image en trois dimensions d'un membre est obtenue.

Par exemple, un membre non-amputé du patient est numérisé en utilisant un scanner 3D pour obtenir ladite image. Par ladite image on obtient une représentation de la surface de ce membre, c'est à dire on obtient une représentation numérique en trois dimensions du membre. La représentation numérique du membre est une surface en trois dimensions.

Alternativement, une image en trois dimensions sortie d'une bibliothèque de données pourrait-être utilisée. Dans ce cas, ladite image est obtenue à partir d'une bibliothèque de morphotypes basés sur la taille et le poids du patient.

On pourrait aussi se servir d'une image scannée du membre du patient faite avant l'amputation du membre.

Dans l'exemple présent on obtient une représentation numérique d'une partie tibiale d'une jambe, c'est à dire une représentation numérique d'une partie inférieure de la jambe tel que montrée aux figures 2 et 4 pour une prothèse de jambe Pour une prothèse de bras inférieur on obtient une représentation numérique du bras inférieur, c'est à dire une représentation numérique d'une partie tel que montrée à la figure 7 en bas.

De la même façon, on peut également obtenir une représentation numérique d'un pied. Comme décrit ci-dessus, il est possible d'utiliser un scanner 3D ou de récupérer une image d'une bibliothèque d'images.

De la même façon, on peut également obtenir une représentation numérique d'une jambe avec son pied, en utilisant un scanner 3D ou en récupérant une image d'une bibliothèque d'images.

De la même façon, on peut également obtenir une représentation numérique d'une main, d'un bras avec sa main ou d'un bras et d'une main séparément.

Dans une deuxième étape, les parties du membre ayant des duretés différentes (7, 8) sont identifiées sur la représentation numérique du membre. Cette identification peut se faire de façon automatique, de façon manuelle par un opérateur ou par un opérateur assisté par un algorithme. Cette identification peut se faire sur la représentation numérique complète ou seulement sur une partie de la représentation numérique. Dans l'exemple d'une représentation numérique d'une jambe avec son pied, cette identification peut se faire, par exemple, seulement sur la partie de la jambe en excluant le pied. A la suite de cette étape, une délimitation des parties ayant des duretés différentes peut être tracée sur la représentation du membre. Autrement dit un agencement des parties de dureté différentes entre elles peut être tracée sur la représentation du membre.

Par exemple, sur une représentation numérique d'une partie tibiale d'une jambe, on identifie la partie correspondant au tibia et la partie correspondant au tibial antérieur. Ensuite, on trace sur la représentation numérique une ligne pour délimiter une extension du tibia et du tibial antérieur sous la peau. Par exemple, sur une représentation numérique d'un bras, on identifie la partie correspondant à l'ulna et la partie correspondant au flexor. Ensuite, on trace sur la représentation numérique une ligne pour délimiter une extension de l'ulna et du flexor sous la peau.

De façon avantageuse, on délimite également la première zone qui va assurer majoritairement la liaison que la structure exosquelettique assure entre l'emboîture et la prothèse de main ou de pied. Par exemple, on trace sur la représentation numérique une zone similaire à la position du tibia ou l'ulna, qui sera sur la structure exosquelettique la première zone pour assurer une transmission de force entre l'emboîture et la prothèse de main ou de pied, afin d'assurer ladite liaison.

Dans une troisième étape, la structure exosquelettique est conçu. La structure exosquelettique est conçu, selon le mode de réalisation, venu de matière avec l'emboîture et/ou la prothèse de pied ou de main ou séparément. La deuxième et la troisième étape peuvent être réalisées en utilisant un logiciel de conception assistée par ordinateur (CAO).

Sur un modèle numérique de la structure exosquelettique, une zone est définie en définissant sa forme. On trace alors sur la surface de la structure exosquelettique une forme de zone. Ensuite, une flexibilité de cette zone est définie. On fixe par exemple l'épaisseur que le matériau de la structure exosquelettique va avoir au sein de cette zone. A la fin de cette étape, un agencement de zones de différentes flexibilités, par exemple obtenu par différentes épaisseurs, est défini sur la structure exosquelettique. De façon avantageuse, la première zone est définie afin de pouvoir assurer majoritairement la liaison entre l'emboîture et la prothèse de pied et de main seule. La forme de la première zone et/ou l'épaisseur de la première zone et/ou le matériau de la première zone est ou sont ainsi choisi pour que la première zone puisse transmettre une force requise entre l'emboîture et la prothèse de main ou de pied. La force requise dépend d'une utilisation prévue. La force requise peut être, par exemple, plus grande pour une prothèse de jambe que pour une prothèse de bras, plus grande pour une prothèse d'adulte que pour une prothèse d'enfant et plus grande pour une prothèse pour un patient ayant un grand poids corporel que pour un patient léger. Le matériau et/ou l'épaisseur et/ou la structure et/ou un renfort et/ou la forme géométrique seront ainsi choisi pour que la première zone puisse transmettre la force requise toute seule, c'est à dire sans une présence d'autres zones.

La forme et la flexibilité des zones sont définies de façon à correspondre à l'agencement des parties du membre ayant des duretés différentes.

Dans un exemple utilisant des épaisseurs différentes, sur la structure exosquelettique, une première zone ayant une épaisseur plus importante sera située par rapport à une deuxième zone ayant une épaisseur moins importante comme une partie du membre plus dure se situe par rapport à une partie moins dure sur la représentation numérique du membre, lesdites parties étant identifiées auparavant comme décrit ci-dessus.

Pour vérifier l'agencement desdites zones entre elles et la correspondance à l'agencement des parties du membre on peut projeter la représentation numérique de la structure exosquelettique pour prothèse sur la représentation numérique du membre.

De cette façon, une zone ayant une épaisseur plus importante qu'une épaisseur moyenne se trouve arrangée pour coïncider avec une partie dure du membre et/ou une zone ayant une épaisseur moins importante qu'une épaisseur moyenne se trouve arrangée pour coïncider avec une partie molle du membre.

Par exemple, on trace sur un modèle numérique de la structure exosquelettique tel qu'on le voit à la figure 1 une première zone (5.1) correspondant par sa forme à la partie (13) du tibia. La partie du tibia a été auparavant identifiée sur la représentation numérique de la jambe.

Ensuite, on trace une deuxième zone (6.1) sur le modèle numérique de la structure exosquelettique correspondant par sa forme à la partie (14) du tibial antérieur, tel que identifiée sur la représentation numérique de la jambe auparavant.

Ensuite, on associe une épaisseur plus importante avec la première zone et une épaisseur moins importante avec la deuxième zone.

La structure exosquelettique fabriqué sera ainsi moins flexible au niveau de la première zone qu'au niveau de la deuxième zone, tel qu'une jambe est plus dure à une partie correspondant au tibia et plus molle à une partie correspondant tibial antérieur. La structure exosquelettique confère de cette façon un toucher physiologique à un utilisateur.

Il est également possible de donner à la structure exosquelettique la forme du membre tel qu'elle est déduite de la représentation numérique du membre d'origine. La structure exosquelettique adopte de cette façon la forme du membre. La structure exosquelettique peut ainsi adopter la forme en trois dimensions de la jambe, par exemple de la partie tibiale ou du bras, par exemple de l'avant-bras. De plus, le pied prothétique peut adopter la forme du pied tel qu'identifié sur la représentation numérique et la main prothétique peut adopter la forme de la main tel qu'identifié sur la représentation numérique.

Une structure exosquelettique qui adopte la forme du membre et confère en même temps un toucher physiologique est particulièrement bien adapté pour fournir à un utilisateur un sentiment naturel quand il porte sa prothèse.

La structure exosquelettique peut ensuite être fabriquée par un procédé d'impression 3D à partir du modèle numérique. Plus particulièrement, on peut utiliser une impression en 3D par fusion de poudre, par exemple en TPU.

Il est ainsi possible de fabriquer une structure exosquelettique pour une prothèse de la jambe ou pour une prothèse du bras.

Dans le cas d'une prothèse de la jambe, la structure exosquelettique comprenant la prothèse de pied peut être fabriquée à partir d'une représentation numérique d'une jambe complète avec son pied, par le procédé décrit ci-dessus. Dans ce cas, il est possible de fabriquer la prothèse comprenant le pied prothétique. La structure exosquelettique pour la prothèse de la jambe et la prothèse du pied peuvent être fabriquées en deux parties séparées. La structure exosquelettique pour la prothèse de la jambe et la prothèse du pied peuvent aussi être fabriqués venu de matière l'une avec l'autre. On obtient dans le dernier cas une prothèse en un seul élément de matière continue comprenant la prothèse du pied et la structure exosquelettique en un seul élément de matière continue. En d'autres mots, à partir d'une représentation numérique d'une jambe complète avec son pied on peut fabriquer la prothèse de la jambe comprenant la prothèse du pied par un seul élément de matière continue. On peut conférer à cette prothèse la forme tel que définie par la représentation numérique. En d'autres mots, la structure exosquelettique reproduit la forme de la jambe à un niveau de mollet selon la représentation numérique et la prothèse du pied reproduit la forme du pied selon la représentation numérique. En résumé, on peut obtenir à partir d'une représentation d'une jambe complète avec son pied une prothèse complète, incluant une partie tibiale avec une prothèse de pied. Cette prothèse peut avoir la forme de la jambe complète avec son pied et conférer, par endroits ou complètement, un toucher physiologique.

De la même façon on peut obtenir une prothèse de membre supérieur, par exemple d'un bras. Dans le cas d'une prothèse du bras, la prothèse peut également être fabriqué à partir d'une représentation numérique d'un bras complet avec sa main, par le procédé décrit ci-dessus. La structure exosquelettique pour la prothèse du bras et la prothèse de la main peuvent être fabriqués en deux parties séparées ou venues de matière l'une avec l'autre. En d'autres mots, il est possible de fabriquer à partir d'une représentation numérique du bras complet avec sa main la structure exosquelettique et la prothèse de la main par un seul élément de matière continue. La structure exosquelettique et/ou la prothèse de main peuvent adopter la forme telle que définie par la représentation numérique du bras avec ou sans sa main.

La figure 11 montre une structure exosquelettique d'une prothèse (1), une emboîture (29), un composant électronique (39) et une fixation pour composant électronique (38). La figure 11 montre également un moteur (40) et une fixation de moteur (41) et un senseur (42). Le composant électronique est fixé à l'intérieur de la structure exosquelettique par la fixation (38). Le moteur est fixé d'un côté opposé à l'emboîture par la fixation de moteur. Le senseur (42) est fixé à l'intérieur de l'emboîture afin de pouvoir rentrer en contact avec un moignon d'un utilisateur.

Dans l'exemple présent, l'emboîture est venue de matière avec la structure exosquelettique. La structure exosquelettique comprenant la fixation peut être conçu et fabriqué par le procédé décrit ci-dessus. Dans ce cas, le procédé peut comprendre une étape durant laquelle l'emplacement de la fixation est déterminé, de préférence de façon à prévenir une influence sur le toucher physiologique conféré par la structure exosquelettique.

La structure exosquelettique montré dans la figure 11 est particulièrement bien adapté pour former une prothèse myoélectrique. Les prothèses myoélectriques fonctionnent grâce aux contractions musculaires contrôlées par le patient. De façon avantageuse le senseur (42) est ainsi un senseur d'une prothèse myoélectrique. Le senseur (42) capte les signaux musculaires du moignon du patient. Les signaux peuvent être amplifiés et ensuite envoyés au moteur (41). Le moteur peut se mettre en action grâce à une énergie fournie par une pile. Le composant électronique (39) peut, par exemple, être une pile ou un processeur conçu pour gérer un fonctionnement du moteur (40) à la base des signaux envoyés par le senseur (42) ou un amplificateur pour amplifier les signaux captés par le senseur (42).

## Revendications

1. Structure exosquelettique, ayant de préférence une forme tubulaire (1), d'une prothèse (12) d'un membre (2) donné, **caractérisée en ce que** la structure exosquelettique est conçue pour assurer une liaison
- entre une emboîture (29) et une prothèse de main (37) ou
- entre une emboîture (29) et une prothèse de pied (25)
afin de former la prothèse du membre,
la structure exosquelettique comprenant au moins deux zones (5.1, 5.2, 6.1, 6.2) de flexibilité différentes (3.1, 3.2, 3.3, 9.1, 9.2),
l'agencement desdites zones entre elles correspondant à l'agencement de parties du membre donné ayant des duretés différentes (7, 8).

2. Structure exosquelettique (1) selon la revendication 1, dans laquelle ladite liaison est assurée majoritairement par la première zone seule.

3. Structure exosquelettique selon la revendication 1 ou 2, dans lequel une première zone (5.1, 5.2) diffère d'une deuxième zone (6.1, 6.2) par rapport à :
- une épaisseur et/ou
- un matériau et/ou
- un motif en trois dimensions
afin d'obtenir une première zone ayant une flexibilité différente d'une deuxième zone.

4. Structure exosquelettique selon la revendication 1, 2 ou 3, dans lequel :
- une première zone a une flexibilité moins (3.1, 3.2, 3.3) importante qu'une deuxième zone, la première zone étant arrangée pour coïncider avec une partie dure (7) du membre, et/ou
- une deuxième zone a une flexibilité plus importante (9.1, 9.2) que une première zone, la deuxième zone étant arrangée pour coïncider avec une partie molle (8) du membre.

5. Structure exosquelettique selon une des revendications 1 à 4, dans lequel :
- une première zone (5.1, 5.2) et une deuxième zone (6.1, 6.2) présentent des épaisseurs différentes, et
- la première zone et la deuxième zone sont obtenues par une nervure (10) ou par une échancrure (11).

6. Structure exosquelettique selon une des revendications 1 à 5, dans lequel la structure exosquelettique a une forme tubulaire et
- une flexibilité de la structure exosquelettique reste majoritairement constante le long d'une longueur de la forme tubulaire, et/ou
- la flexibilité de la structure exosquelettique change majoritairement le long d'une circonférence de la forme tubulaire.

7. Structure exosquelettique d'une prothèse de bras selon une des revendications précédentes en combinaison avec la revendication 3, dans laquelle:
- une zone ayant une épaisseur plus importante (3.1, 3.2, 3.3) qu'une épaisseur moyenne de la structure exosquelettique est située sur la structure exosquelettique comme le radius ou ulna (40, 41) est situé sur un bras et/ou
- une zone ayant une épaisseur moins importante (6.1, 6.2) qu'une épaisseur moyenne de la structure exosquelettique est située sur la structure exosquelettique comme un muscle (15) ou un tendon (16) est situé sur un bras.

8. Structure exosquelettique selon une des revendications précédentes en combinaison avec la revendication 3, dans lequel une surface extérieure (21) de la structure exosquelettique est majoritairement lisse et les changements d'épaisseur sont situés à une surface intérieure (22) de la structure exosquelettique.

9. Structure exosquelettique selon une des revendications précédentes, dans lequel la structure exosquelettique comprend à un intérieur de la structure exosquelettique au moins une fixation (38) conçue pour fixer un composant électronique (39).

10. Structure exosquelettique selon une des revendications précédentes, dans lequel :
- un matériau de la structure exosquelettique comprend un polyuréthane thermoplastique (TPU) et/ou
- un matériau de la structure exosquelettique comprend un polyamide.

11. Prothèse d'un membre donné comprenant :
- une structure exosquelettique selon une des revendications 1 à 10 et
- une emboîture (29) et
- une prothèse de pied (25) ou une prothèse de main (37),
la structure exosquelettique étant situé :
- entre l'emboîture et la prothèse de pied ou
- entre l'emboîture et la prothèse de main
afin d'assurer ladite liaison, de préférence dans laquelle la structure exosquelettique est venu de matière avec l'emboîture pour former un élément de matière continue et/ou la structure exosquelettique est venu de matière avec la prothèse de pied ou de main pour former un élément de matière continue.

12. Procédé de fabrication d'une structure exosquelettique selon l'une des revendications 1 à 10, le procédé comprenant les étapes suivantes :
- obtenir une image en trois dimensions d'un membre,
- identifier sur l'image les parties du membre ayant des duretés différentes (7, 8),
- définir sur un modèle numérique de la structure exosquelettique des zones de flexibilité différentes (5.1, 5.2, 6.1, 6.2) de façon à correspondre à l'agencement desdites parties identifiées sur ladite image,
- réalisation de la structure exosquelettique à partir du modèle numérique.

13. Procédé de fabrication d'une prothèse selon la revendication 11, comprenant les étapes du procédé de fabrication d'une structure exosquelettique selon la revendication 12, dans lequel
- l'image en trois dimensions est obtenue à partir d'une jambe complète avec son pied ou à partir d'un bras complet avec sa main.

14. Procédé selon la revendication 12 ou 13 comprenant l'étape suivante :
- identifier sur l'image la forme du membre
- définir le modèle numérique de la structure exosquelettique de façon à ce que la forme de la structure exosquelettique corresponde à la forme du membre telle qu'identifiée sur l'image.

15. Procédé selon la revendication 12, 13 ou 14, dans lequel l'étape de réalisation est une étape d'impression en 3D, de préférence par fusion de poudre, de la structure exosquelettique à partir dudit modèle numérique.

## Patentansprüche

1. Exoskelettstruktur, die vorzugsweise eine röhrenförmige Form (1) aufweist, einer Prothese (12) einer jeweiligen Gliedmaße (2), **dadurch gekennzeichnet, dass** die Exoskelettstruktur so ausgelegt ist, dass sie eine Verbindung
- zwischen einer Aufnahme (29) und einer Handprothese (37) oder
- zwischen einer Aufnahme (29) und einer Fußprothese (25), um die Prothese der Gliedmaße zu bilden, gewährleistet,
wobei die Exoskelettstruktur mindestens zwei Bereiche (5.1, 5.2, 6.1, 6.2) unterschiedlicher Flexibilität (3.1, 3.2, 3.3, 9.1, 9.2) umfasst,
wobei die Anordnung der Bereiche untereinander, die der Anordnung von Teilen der jeweiligen Gliedmaße mit unterschiedlichen Härten (7, 8) entspricht.

2. Exoskelettstruktur (1) nach Anspruch 1, wobei die Verbindung überwiegend durch den ersten Bereich allein gewährleistet ist.

3. Exoskelettstruktur nach Anspruch 1 oder 2, wobei sich ein erster Bereich (5.1, 5.2) von einem zweiten Bereich (6.1, 6.2) unterscheidet in Bezug auf:
- eine Dicke und/oder
- ein Material und/oder
- ein dreidimensionales Muster, um einen ersten Bereich mit einer anderen Flexibilität als einen zweiten Bereich zu erhalten.

4. Exoskelettstruktur nach Anspruch 1, 2 oder 3, wobei:
- ein erster Bereich eine geringere Flexibilität (3.1, 3.2, 3.3) als ein zweiter Bereich aufweist, wobei der erste Bereich so angeordnet ist, dass er mit einem harten Teil (7) der Gliedmaße zusammenfällt, und/oder
- ein zweiter Bereich eine größere Flexibilität (9.1, 9.2) als ein erster Bereich aufweist, wobei der zweite Bereich so angeordnet ist, dass er mit einem weichen Teil (8) der Gliedmaße zusammenfällt.

5. Exoskelettstruktur nach einem der Ansprüche 1 bis 4, wobei:
- ein erster Bereich (5.1, 5.2) und ein zweiter Bereich (6.1, 6.2) unterschiedliche Dicke aufweisen, und
- der erste Bereich und der zweite Bereich durch eine Rippe (10) oder eine Aussparung (11) erhalten werden.

6. Exoskelettstruktur nach einem der Ansprüche 1 bis 5, wobei die Exoskelettstruktur eine röhrenförmige Form aufweist und
- eine Flexibilität der Exoskelettstruktur entlang einer Länge der röhrenförmigen Form überwiegend konstant bleibt, und/oder
- die Flexibilität der Exoskelettstruktur sich entlang eines Umfangs der röhrenförmigen Form überwiegend ändert.

7. Exoskelettstruktur einer Armprothese nach einem der vorhergehenden Ansprüche in Kombination mit Anspruch 3, wobei:
- ein Bereich mit einer größeren Dicke (3.1, 3.2, 3.3) als eine durchschnittliche Dicke der Exoskelettstruktur auf der Exoskelettstruktur liegt, wie der Radius oder die Ulna (40, 41) auf einem Arm liegt und/oder
- ein Bereich mit einer geringeren Dicke (6.1, 6.2) als eine durchschnittliche Dicke der Exoskelettstruktur auf der Exoskelettstruktur liegt, wie ein Muskel (15) oder eine Sehne (16) auf einem Arm liegt.

8. Exoskelettstruktur nach einem der vorhergehenden Ansprüche in Kombination mit Anspruch 3, wobei eine Außenfläche (21) der Exoskelettstruktur überwiegend glatt ist und die Dickenänderungen an einer Innenfläche (22) der Exoskelettstruktur liegen.

9. Exoskelettstruktur nach einem der vorhergehenden Ansprüche, wobei die Exoskelettstruktur an einer Innenseite der Exoskelettstruktur mindestens eine Befestigung (38) zum Befestigen einer elektronischen Komponente (39) umfasst.

10. Exoskelettstruktur nach einem der vorhergehenden Ansprüche, wobei:
- ein Material der Exoskelettstruktur ein thermoplastisches Polyurethan (TPU) umfasst und/oder
- ein Material der Exoskelettstruktur ein Polyamid umfasst.

11. Prothese einer jeweiligen Gliedmaße, umfassend:
- eine Exoskelettstruktur nach einem der Ansprüche 1 bis 10 und
- eine Aufnahme (29) und
- eine Fußprothese (25) oder eine Handprothese (37), wobei sich die Exoskelettstruktur befindet:
- zwischen der Aufnahme und der Fußprothese oder
- zwischen der Aufnahme und der Handprothese, um die Verbindung zu gewährleisten, wobei die Exoskelettstruktur vorzugsweise aus Material mit der Aufnahme stammt, um ein Element aus durchgehendem Material zu bilden, und/oder die Exoskelettstruktur aus Material mit der Fuß- oder Handprothese stammt, um ein Element aus durchgehendem Material zu bilden.

12. Verfahren zur Herstellung der Exoskelettstruktur nach einem der Ansprüche 1 bis 10, wobei das Verfahren die folgenden Schritte umfasst:
- Erhalten eines dreidimensionales Bildes einer Gliedmaße,
- Identifizieren, auf dem Bild, der Teile der Gliedmaße mit unterschiedlichen Härten (7, 8),
- Definieren verschiedener Flexibilitätsbereichen (5.1, 5.2, 6.1, 6.2) auf einem digitalen Modell der Exoskelettstruktur, so dass sie der Anordnung der auf dem Bild identifizierten Teile entsprechen,
- Ausführen der Exoskelettstruktur anhand des digitalen Modells.

13. Verfahren zur Herstellung einer Prothese nach Anspruch 11, umfassend die Verfahrensschritte zur Herstellung einer Exoskelettstruktur nach Anspruch 12, wobei
- das dreidimensionale Bild von einem kompletten Bein mit dem Fuß oder von einem kompletten Arm mit der Hand erstellt wird.

14. Verfahren nach Anspruch 12 oder 13, umfassend folgenden Schritt:
- Identifizieren der Form der Gliedmaße auf dem Bild
- Definieren des digitalen Modells der Exoskelettstruktur, so dass die Form der Exoskelettstruktur der Form der Gliedmaße entspricht, wie auf dem Bild identifiziert.

15. Verfahren nach Anspruch 12, 13 oder 14, wobei der Ausführungsschritt ein 3D-Druckschritt, vorzugsweise durch Pulverschmelzen, der Exoskelettstruktur aus dem genannten digitalen Modell ist.

## Claims

1. Exoskeletal structure, having preferably a tubular shape (1), of a prosthesis (12) of a given limb (2), **characterized in that** the exoskeletal structure is designed to provide a connection
- between a socket (29) and a hand prosthesis (37) or
- between a socket (29) and a foot prosthesis (25)
in order to form the prosthesis of the limb,
the exoskeletal structure comprising at least two zones (5.1, 5.2, 6.1, 6.2) of different flexibility (3.1, 3.2, 3.3, 9.1, 9.2),
the arrangement of said zones in relation to one another corresponding to the arrangement of the parts of the given limb having different hardnesses (7, 8).

2. Exoskeletal structure (1) according to claim 1, wherein said connection is provided mostly by the first zone alone.

3. Exoskeletal structure according to claim 1 or 2, wherein a first zone (5.1, 5.2) differs from a second zone (6.1, 6.2) in relation to:
- a thickness and/or
- a material and/or
- a three-dimensional pattern
in order to obtain a first zone having a different flexibility from a second zone.

4. Exoskeletal structure according to claim 1, 2 or 3, wherein:
- a first zone has a lesser flexibility (3.1, 3.2, 3.3) than a second zone, the first zone being arranged to coincide with a hard part (7) of the limb, and/or
- a second zone has a greater flexibility (9.1, 9.2) than a first zone, the second zone being arranged to coincide with a soft part (8) of the limb.

5. Exoskeletal structure according to one of claims 1 to 4, wherein:
- a first zone (5.1., 5.2) and a second zone (6.1, 6.2) have different thicknesses, and
- the first zone and the second zone are obtained with a rib (10) or with a recess (11).

6. Exoskeletal structure according to one of claims 1 to 5, wherein the exoskeletal structure has a tubular shape and
- a flexibility of the exoskeletal structure remains mostly constant along a length of the tubular shape, and/or
- the flexibility of the exoskeletal structure mostly changes along a circumference of the tubular shape.

7. Exoskeletal structure of an arm prosthesis according to one of the preceding claims in combination with claim 3, wherein:
- a zone having a greater thickness (3.1, 3.2, 3.3) than a mean thickness of the exoskeletal structure is located on the exoskeletal structure like the radius or ulna (40, 41) is located on an arm and/or
- a zone having a lesser thickness (6.1, 6.2) than a mean thickness of the exoskeletal structure is located on the exoskeletal structure like a muscle (15) or a tendon (16) is located on an arm.

8. Exoskeletal structure according to one of the preceding claims in combination with claim 3, wherein an outer surface (21) of the exoskeletal structure is mostly smooth and the changes in thickness are located on an inner surface (22) of the exoskeletal structure.

9. Exoskeletal structure according to one of the preceding claims, wherein the exoskeletal structure comprises at an interior of the exoskeletal structure at least one attachment device (38) designed to attach an electronic component (39).

10. Exoskeletal structure according to one of the preceding claims, wherein:
- a material of the exoskeletal structure comprises a thermoplastic polyurethane (TPU) and/or
- a material of the exoskeletal structure comprises a polyamide.

11. Prosthesis of a given limb comprising:
- an exoskeletal structure according to one of claims 1 to 10 and
- a socket (29) and
- a foot prosthesis (25) or a hand prosthesis (37),
the exoskeletal structure being located:
- between the socket and the foot prosthesis or
- between the socket and the hand prosthesis
in order to provide said connection, preferably wherein the exoskeletal structure is integral with the socket to form a continuous material element and/or the exoskeletal structure is integral with the foot or hand prosthesis to form a continuous material element.

12. Method for manufacturing an exoskeletal structure according to one of claims 1 to 10, the method comprising the following steps:
- obtaining a three-dimensional image of a limb,
- identifying in the image the parts of the limb having different hardnesses (7, 8),
- defining on a digital model of the exoskeletal structure zones of different flexibility (5.1, 5.2, 6.1, 6.2) so as to correspond to the arrangement of said parts identified in said image,
- producing the exoskeletal structure from the digital model.

13. Method for manufacturing a prosthesis according to claim 11, comprising the steps of the method for manufacturing an exoskeletal structure according to claim 13, wherein
- the three-dimensional image is obtained from a complete leg with its foot or from a complete arm with its hand.

14. Method according to claim 12 or 13 comprising the following step:
- identifying in the image the shape of the leg
- defining the digital model of the exoskeletal structure such that the shape of the exoskeletal structure corresponds to the shape of the limb as identified in the image.

15. Method according to claim 12, 13 or 14, wherein the production step is a step of 3D printing, preferably using powder melting, of the exoskeletal structure from said digital model.
